# EUROPÄISCHE PATENTSCHRIFT

(11) **EP 1 920 739 B1**
(45) Veröffentlichungstag und Bekanntmachung des Hinweises auf die Patenterteilung: **07.12.2011**
(21) Anmeldenummer: 07079506.7
(22) Anmeldetag: 07.09.2007
(51) Int. Cl.: A61F 2/84

(54) **Stenteinführsystem**
Stent insertion system
Système d'introduction d'un stent

(30) Priorität: 09.11.2006 DE 102006053748
(43) Veröffentlichungstag der Anmeldung: 14.05.2008
(73) Patentinhaber: JOTEC GmbH, 72379 Hechingen (DE)
(72) Erfinder: Barthold, Franz-Peter, 72336 Balingen (DE); Lesmeister, Rainer, 72770 Reutlingen (DE)
(74) Vertreter: Laufer, Gabriele

(56) Entgegenhaltungen:
- EP-A- 1 369 098
- EP-A- 1 440 673
- WO-A-2005/023149

## Beschreibung

Die vorliegende Erfindung betrifft ein Einführsystem zum Einführen und Freisetzen eines selbstexpandierenden Stents in ein Körpergefäß, mit einem selbstexpandierenden Stent, der einen hohlzylindrischen Körper mit einem proximalen und einem distalen Ende aufweist, wobei zumindest das proximale Ende abwechselnd in proximale und distale Richtung weisende Spitzbögen aufweist, die Scheitel und gerade Abschnitte aufweisen.

Gefäßstents, die auch als endovaskuläre Stents bezeichnet werden, werden zur Freihaltung eines Gefäßes in dieses eingeführt, und sind im Stand der Technik ausführlich beschrieben. So können beispielsweise Gefäßstentgrafts in verletzte oder verschlossene Gefäße eingeführt werden, um die Gefäßwände an den verletzten oder verschlossenen Stellen zu ersetzen. Ein Stentgraft weist hierfür neben einem Grundgerüst, das meistens aus Draht ist, einen Mantel aus biokompatiblen Material auf.

Im Stand der Technik bekannte Stents werden beispielsweise in Gefäße eingesetzt, deren Wände krankhaft oder durch Verletzung ausgedünnt oder verdickt sind, und die eine dahingehende Unterstützung benötigen.

Viele Stents stellen selbstexpandierende Stentsysteme dar, die in komprimiertem Zustand in das Gefäß eingeführt werden, und deren Expandierung durch Entfernen von komprimierenden Mitteln ermöglicht wird. Bei diesen selbstexpandierenden Stents ist daher notwendig, dass sie ein elastisches Material aufweisen, das sich nach außen, d.h. radial expandieren kann, sobald eine zur Kompression auf das Material ausgeübte Kraft, bspw. eine Hülle, entfernt wird. Hier wird vorzugsweise Nitinol verwendet, das darüber hinaus auch Shape-memory-Eigenschaften aufweisen kann. Das Stentgerüst wird aus diesem Material gefertigt und weist vorzugsweise eine röhrenförmige Struktur auf, die meist einen etwas größeren Durchmesser als das Gefäß aufweist, in das es denn eingebracht werden soll.

Die Einführung und Einbringung eines Stents oder Stentgrafts wird meist unter Einsatz eines Einführungssystems durchgeführt, bei welchem zwei röhrenförmige Strukturen vorzusehen sind, nämlich ein innerer Kolben und eine äußere Hülle, die relativ zueinander axial bewegt werden können. Der Stent ist dabei innerhalb des distalen Endes der äußeren Hülle im komprimiertem Zustand angeordnet und wird in diesem Zustand in das Gefäß eingeführt. Nach Platzierung des Stents an der gewünschten Stelle wird der Kolben meist stationär gehalten, wohingegen die Hülle des Einführungssystems zurückgezogen wird, wodurch der Stent freigesetzt wird. Dieser kann durch Anschlagen an den Kolben sich nicht in Richtung der zurückgezogenen Hülle bewegen, wenn diese entfernt wird. Der Stent entfaltet sich aufgrund seiner selbstexpandierenden Eigenschaften und legt sich an die Gefäßwände an.

Im Stand der Technik wird allgemein das Ende des Stents, das näher zu dem Herzen platziert wird, als proximales Ende bezeichnet, wohingegen das Ende des Stents, das weiter weg vom Herzen platziert wird, als distales Ende bezeichnet wird. Im Gegensatz hierzu werden die Enden des Einführsystems dahingehend mit distal und proximal bezeichnet, dass das Ende, das näher bei der handhabenden Person liegt, als proximal bezeichnet wird, und das Ende, das weiter entfernt ist von der handhabenden Person als das andere Ende, als distal bezeichnet wird.

Das proximale Ende des Stents bzw. Stentgrafts ist typischerweise derart ausgestaltet, dass der Stent vor allem mit diesem Ende an der Gefäßwand fixiert wird. Dies soll ein Verrutschen des Stents nach dessen Einbringung in das Gefäß verhindern. Die Enden des Stents weisen dabei Federelemente auf, die mäanderförmige umlaufende Spitzbögen bilden, und die sich nach deren Freisetzung radial expandieren und an der Gefäßwand als Fixierelemente dienen. Das proximale Ende mit diesen Fixierelementen bzw. Spitzbögen weist im expandierten Zustand meist einen größeren Durchmesser auf als das Gefäß, in das der Stent eingebracht wurde, gerade damit sich zumindest die Spitzbögen an dem proximalen Stentende fest an die Gefäßwände anlagern und dort verankern können. Daher müssen diese Spitzbögen am proximalen Stentende zur Einbringung des Stents zusammengedrückt bzw. komprimiert und im Einführungssystem lösbar befestigt werden.

Bei vielen im Stand der Technik bekannten Stents ist neben den restlichen Teilen des Stents auch das proximale Ende durch den Hüllschlauch komprimiert, der den Stent zur Einführung in das Gefäß im komprimierten Zustand hält.

Im Stand der Technik sind auch Einführsysteme bekannt, die eine getrennte Freisetzung des proximalen Stentendes vom restlichen Stent ermöglichen.

Dabei ist es oftmals ein Problem, dass es beim Einführen des Systems und des Stents notwendig ist, das System vor der Expandierung leicht zurückzuziehen, da der Stent bzw. das System im Gefäß zu weit vorgeschoben war. Bei diesem Zurückziehen weisen viele Stents und Einführsysteme des Standes der Technik den Nachteil auf, dass sich dann die Spitzbögen des proximalen Endes des Stents in der Gefäßwand verhaken.

Zur Lösung dieses Problems ist im Stand der Technik beispielsweise das Einführsystem aus der EP 1 369 098 A1 bekannt, bei dem eine Kappe vorgesehen ist, innerhalb welcher die proximalen Federenden des Stents geführt und befestigt sind, und die durch einen Mechanismus in der Kappe bzw. durch Entfernen der Kappe freigesetzt werden.

Aus der WO 2005/023149 ist ein Einführsystem für Stents bzw. Stentgrafts bekannt, bei welchem die Federn des Stents durch eine Einfangvorrichtung für die Spitzen der Federn des Stents zusammengeführt werden. Diese Einfangvorrichtung weist eine entsprechende Anzahl von fixierten länglichen Fortsätzen auf, in die die Federn eingefädelt werden. Die Fortsätze sind dabei fest auf einem äußeren Rohr bzw. Katheter fixiert, welcher durch das Stentlumen hindurchgeführt ist. Durch Zurückziehen des Rohrs, d.h. durch ein Ziehen des Rohrs in Richtung des Anwenders, sowie der auf diesem fest fixierten Fortsätze werden die eingefädelten Federn des proximalen Stentendes freigesetzt.

Nachteilig bei den im Stand der Technik bekannten Systemen ist, dass bspw. die Gefahr besteht, dass - wenn die Federn sich mit dem sie fixierenden System verhaken, bzw. wenn durch das Rückziehen der Einfangvorrichtung die Federn sich nicht vollständig hiervon lösen - das proximale Ende des Stents nicht freigesetzt werden kann. Daneben ist bei dem aus der WO 2005/023149 bekannten System nachteilig, dass durch ein Verdrehen des äußeren Katheters die Gefahr auch einer Verdrehung des Stents besteht.

Aufgabe der vorliegenden Erfindung ist es daher, ein Einführsystem bereitzustellen, mit welchem die im Stand der Technik bekannten Nachteile überwunden werden können.

Erfindungsgemäß wird diese Aufgabe gelöst durch eine Weiterbildung des eingangs genannten Einführsystems, das ein äußeres Rohr aufweist, das innerhalb des Stent geführt ist, und ein inneres Rohr, das innerhalb des äußeren Rohrs geführt ist, wobei das innere und das äußere Rohr gegeneinander axial verschiebbar angebracht sind, und das ein Fixiersystem für die Spitzbögen des proximalen Stentendes zur Einführung des Stents in das Körpergefäß aufweist, wobei das Fixiersystem ein Deckelelement mit daran befestigten Stiftelementen aufweist, die sich axial und in proximaler Richtung des Einführsystems erstrecken, und ein erstes Aufnahmeelement für die Stiftelemente, das proximal zu dem Deckelelement im Einführsystem angeordnet ist, und wobei das Deckelelement fest mit dem inneren Rohr verbunden ist, und zwar derart, dass durch eine Verschiebung des inneren Rohres in distale Richtung im Verhältnis zum Anwender, die Spitzbögen des proximalen Stentendes aus dem Fixiersystem freisetzbar und in einen expandierten Zustand überführbar sind.

Die der Erfindung zugrunde liegende Aufgabe wird auf diese Weise vollkommen gelöst.

Mit dem erfindungsgemäßen Einführsystem, und insbesondere mit dem darin enthaltenen Fixiersystem mit Deckelelement und Aufnahmeelement für das proximale Stentende ist es nun möglich, die Spitzbögen freizusetzen, ohne dass die Gefahr besteht, dass sich diese im Freisetzungssystem verkanten. Ferner besteht ein weiterer Vorteil darin, dass sämtliche Teile, also u.a. Spitzbögen, Stent und Fixiersystem, gegen eine Rotation gegeneinander gesperrt sind, so dass mit dem Einführsystem Drehmomente übertragen werden können. Damit wird vorteilhafterweise erreicht, dass im Falle einer anfänglich ungenauen Platzierung des Stents dieser um die eigene Achse gedreht werden kann, um den Stent im Gefäß korrekt zu platzieren. Hierbei werden dann die Stentelemente nicht gegeneinander gedreht, da mit der Fixierung der Spitzbögen des proximalen Endes des Stents über die am Deckelelement befestigten Stiftelemente die Sperrung gegen die Rotation gewährleistet wird.

Die Stitftelemente sind dabei vorzugsweise auf den Umfang des Deckelelements kreisförmig verteilt.

Vorliegend werden als "Rohre" alle geeignete Maßnahmen verstanden, die die Eigenschaften eines rohr- oder schlauchförmigen Hohlkörpers aufweisen, wie bspw. ein Katheter.

Vorliegend wird mit "Spitzbögen" jede maschenförmige Struktur eines proximalen Stentendes verstanden, die bogenförmige Abschnitte am äußersten Stentende aufweist, sowie Abschnitte, die im Vergleich zu den bogenförmigen Abschnitten gerader verlaufen. Es versteht sich daher, dass sowohl Stents als auch Stentgrafts mit dem erfindungsgemäßen Einführsystem in ein Körpergefäß eingebracht werden können.

Bei dem erfindungsgemäßen Einführsystem wird also der Stent zur Einführung in ein Gefäß an seinem proximalen Ende über die Stiftelemente des Fixiersystems in der Einführvorrichtung fixiert. Dabei greift jeweils ein Stiftelement in einen Spitzbogen ein, und fädelt dieses sozusagen auf. Die Stitftelemente werden weiter in das Aufnahmeelement geführt, wodurch die Spitzbögen zwischen Deckelelement und Aufnahmeelement fixiert werden. Wenn der Stent im Gefäß richtig platziert ist, wird das innere Rohr gegen das äußere Rohr, auf dem der Stent gelagert ist, in distale Richtung, also vom Anwender weg, bewegt. Dadurch werden die Stiftelemente zunächst aus dem Aufnahmeelement und anschließend aus den Spitzbögen herausgezogen, wodurch diese frei werden und sich radial in Richtung Gefäßwand expandieren. Dadurch weitet sich das proximale Stentende kragenförmig auf und weist in seinem expandierten Endzustand einen Durchmesser auf, der deutlich größer ist als die Elemente des Fixiersystems, so dass in nachfolgenden Schritten, bspw. nach der kompletten Stentfreisetzung, das Fixiersystem durch den Stent zurück, d.h. in Richtung des Anwenders, aus dem Gefäß herausgezogen werden kann.

In einer weiteren Ausführungsform des Einführsystems ist bevorzugt, wenn das erste Aufnahmeelement für die in die proximale Richtung des Einführsystems weisenden Stiftelemente fest mit dem äußeren Rohr verbunden ist, derart, dass das erste Aufnahmeelement und das Deckelelement axial verschiebbar gegeneinander angeordnet sind.

Diese Maßnahme hat den Vorteil, dass während des Freisetzens der Spitzbögen keine Relativbewegung gegen den Stent stattfindet. Zur Freisetzung des Stents wird also das innere Rohr distal, also vom Anwender weg, bewegt, wobei das äußere Rohr feststehend bleibt. Das mit dem inneren Rohr fest verbundene Deckelelement sowie die mit diesem verbundenen Stiftelemente bewegen sich dadurch ebenfalls in distale Richtung und werden zunächst aus dem mit dem inneren Rohr verbundenen Aufnahmeelement und nachfolgend aus den Spitzbögen sozusagen herausgefädelt. Der restliche Teil des Stents bleibt von der Freisetzungsbewegung des proximalen Stentendes unberührt. Durch die Führung der Stiftelemente wird gleichzeitig gewährleistet, dass sich das proximale Stentende nicht gegen den restlichen Teil des Stents bewegt.

Die Stiftelemente sind dabei vorzugsweise aus einem Metall gefertigt, bzw. weisen ein Metall auf, vorzugsweise ein Metall mit Memory-Effekt.

In einer weiteren Ausführungsform ist bevorzugt, wenn das erste Aufnahmeelement zumindest eine Bohrung zur Aufnahme der Stiftelemente aufweist.

Diese Maßnahme hat den Vorteil, dass die Stiftelemente einfach in das Aufnahmeelement eingeführt werden können. Durch eine entsprechende Längenbegrenzung der Stiftelemente kann gewährleistet werden, dass die Stifte in dem Aufnahmeelement enden. Die Bohrungen sind dabei parallel zur Längsachse des Einführsystems und damit auch parallel zum Verlauf der Stiftelemente vorgesehen. Die Bohrung kann dabei durch das Aufnahmeelement hindurchreichen, d.h. von dem Ende des Aufnahmeelements, das dem Deckelelement zugewandt ist, bis zu dem Ende, das dem Deckelelement abgewandt ist. Dabei ist bevorzugt, wenn die Anzahl der Bohrungen der Anzahl der Stiftelemente entspricht.

In einer weiteren Ausführungsform ist vorgesehen, dass das erste Aufnahmeelement längliche, in axiale Richtung zum Einführsystem verlaufende Vertiefungen auf dessen Oberfläche zur Aufnahme der gerade Abschnitte der Spitzbögen aufweist.

Diese Ausführungsform hat den Vorteil, dass die geraden Abschnitte der Spitzbögen des proximalen Stentendes sich in die Vertiefungen einfügen können, und dadurch nicht als Erhebungen auf der Oberfläche des Aufnahmeelements auftreten. Damit wird eine geringere Reibung gewährleistet.

In einer Weiterbildung des erfindungsgemäßen Einführsystems ist bevorzugt, wenn das erste Aufnahmeelement eine Form aufweist, mit einem ersten, dem Deckelelement zugewandten Ende und einem zweiten dem Deckelelement abgewandten Ende, wobei zumindest das erste Ende des ersten Aufnahmeelements einen kleineren Durchmesser aufweist als ein zwischen dem ersten und dem zweiten Ende gelegenes Mittelteil.

Diese Maßnahme hat den Vorteil, dass dadurch das Aufnahmeelement in dessen mittleren Bereich eine sozusagen nach außen gewölbte Struktur aufweist, über die das proximale Stentende geführt wird. Im anschließenden Bereich des Aufnahmeelements, das dem Deckelelement zugewandt ist, werden die Spitzbögen aufgrund des kleineren Durchmessers dieses Endes wieder zusammengeführt und mit Hilfe der Stiftelemente, die in das Aufnahmeelement eingeschoben werden aufgefädelt fixiert.

In einer Weiterbildung es erfindungsgemäßen Einführsystems weist das Fixiersystem ferner ein axial zwischen Deckelelement und Aufnahmeelement gelegenes zweites Aufnahmeelement mit durchgehenden Bohrungen zur lösbaren Durchführung der Stiftelemente auf, welches in axialer Distanz zum ersten Aufnahmeelement mit dem äußeren Rohr fest verbunden ist, derart, dass das mit dem äußeren Rohr fest verbundene erste und zweite Aufnahmeelement gegen das mit dem inneren Rohr fest verbundene Deckelelement axial verschiebbar ist.

Diese Maßnahme hat den Vorteil, dass die Führung der Stifte gewährleistet ist und die Enden der Stifte, wenn sie aus dem Aufnahmeelement herausgezogen werden, nur kurz zur Freigebung der Spitzbögen frei werden. Durch das zweite Aufnahmeelement ist also zusätzlich zur Sicherung der Führung der Stiftelemente während des Freisetzens gesichert, dass die Enden der Stifte nicht frei liegen, und sich damit auch nicht im Gefäß oder sonstigen Bestandteilen des Einführsystems verkanten oder verhaken können.

Das zweite Aufnahmeelement kann dabei bspw. auf seiner Oberfläche längliche, in axiale Richtung zum Einführsystem verlaufende Vertiefungen und zwischen diese gelegene Erhebungen aufweisen.

Dieses Merkmal hat den Vorteil, dass in den Erhebungen die Bohrungen vorliegen können, durch welche die Stiftelemente hindurchgeführt werden, so dass weniger Material zur Fertigung benötigt wird und die Reibungsoberfläche vermindert wird.

Ferner ist insgesamt bevorzugt, wenn die Anzahl der Stiftelemente der Anzahl der Spitzbögen des proximalen Stentendes entspricht.

Dabei wird also jeder Spitzbogen, der am proximalen Stentende vorgesehen ist in ein entsprechendes Stiftelement eingefädelt. Dementsprechend kann dann jedes Stiftelement, das durch das zweite Aufnahmeelement hindurch geführt ist, in eine entsprechende Bohrung des ersten Aufnahmeelements eingeführt werden. Insgesamt ist bevorzugt, wenn mindestens zwei Stiftelemente zur Fixierung von mindestens zwei Spitzbögen vorgesehen sind. Es versteht sich, dass das erfindungsgemäße Einführsystem, insbesondere dessen Fixiersystem, den unterschiedlichen Stents bzw. Stentenden angepasst werden kann, und dass daher die Anzahl der Stifte in Abhängigkeit der Anzahl der Spitzbögen variiert. So kann das erfindungsgemäße System bspw. auf Stents mit drei, vier, fünf, sechs oder mehr Spitzbögen am proximalen Ende angepasst sein.

In einer weiteren Ausführungsform ist bevorzugt, wenn es ferner eine konisch geformte Spitze aufweist, in der das Deckelelement des Fixiersystems gelagert ist.

Diese Maßnahme hat den Vorteil, dass gleichzeitig die Spitze, die allgemein bei einem Stenteinführsystem vorgesehen ist, gleichzeitig zur Aufnahme des Deckelelements eingesetzt werden kann. Das Deckelsystem mit seinen in proximale Richtung weisenden Stiftelementen ist dabei fest in die Spitze integriert und bewegt sich durch Verschieben des inneren Rohres mit dieser.

In einer weiteren bevorzugten Ausführungsform des erfindungsgemäßen Einführsystems ist ferner eine Rückzugshülle vorgesehen, die den Stent zur Einführung in ein Körpergefäß in einem komprimierten Zustand hält.

Wie weiter oben erwähnt, kann über das Fixiersystem gezielt das proximale Stentende freigesetzt werden. Die restlichen Stentabschnitte können dabei unabhängig von dem proximalen Stentende zur Einführung des Stents in ein Körpergefäß durch Krafteinwirkung einer über den Stent gezogenen Hülle komprimiert und durch Rückziehen der Hülle freigesetzt werden. Dadurch kann der Stent vollständig expandieren und sich im Gefäß verankern.

Ferner ist bei einer bevorzugten Ausführungsform vorgesehen, wenn ein Schiebeelement vorgesehen ist, durch welches das distale Ende und der zwischen distalem und proximalen Ende liegende Teil des Stents in Verbindung mit einem Rückziehen der Rückzugshülle freisetzbar ist.

Mit diesem Schiebeelement, der auch Pusher genannt wird, kann wird also der Kraft, die durch Entfernen der Hülle auf den Stent ausgeübt wird, entgegengewirkt werden. Damit wird erreicht, dass sich der Stent nicht mit der Rückzugshülle in proximale Richtung bewegt, sondern an der Stelle gehalten werden kann, an der er ursprünglich zur Freisetzung vorgesehen war.

Ferner ist bevorzugt, wenn das Einführsystem entweder insgesamt oder aber nur Teile davon aus einem oder mehreren röntgendichten Materialien hergestellt sind.

Es versteht sich, dass die vorstehend genannten und nachstehend noch zu erläuternden Merkmale nicht nur in der jeweils angegebenen Kombination, sondern auch in Alleinstellung oder anderen Kombinationen einsetzbar sind, ohne den Rahmen der vorliegenden Erfindung zu verlassen.

Die Erfindung wird nachstehend durch die Beschreibung und die beigefügten Figuren näher erläutert. Es zeigen:
- Fig. 1a:: eine seitliche Draufsicht auf den distalen Abschnitt einer Ausführungsform des erfindungemäßen Einführsystems;
- Fig. 1b:: einen Längsschnitt durch die in Fig. 1a gezeigte Ausführungsform des erfindungemäßen Einführsystems;
- Fig. 1c:: eine weitere Ansicht der Ausführungsform des erfindungsgemäßen Einführsystems aus Fig. 1a, aus seitlicher Vorderansicht;
- Fig. 2:: einen beispielhaften Stent in seiner expandierten Form;
- Fig. 3:: eine seitliche Draufsicht auf den distalen Abschnitt einer Ausführungsform des mit einem Stent geladenen erfindungemäßen Einführsystems, wobei hier das proximale Stentende durch das Fixiersystem der erfindungsgemäßen Ausführungsform zur Einführung des Stents in ein Gefäß fixiert wird.

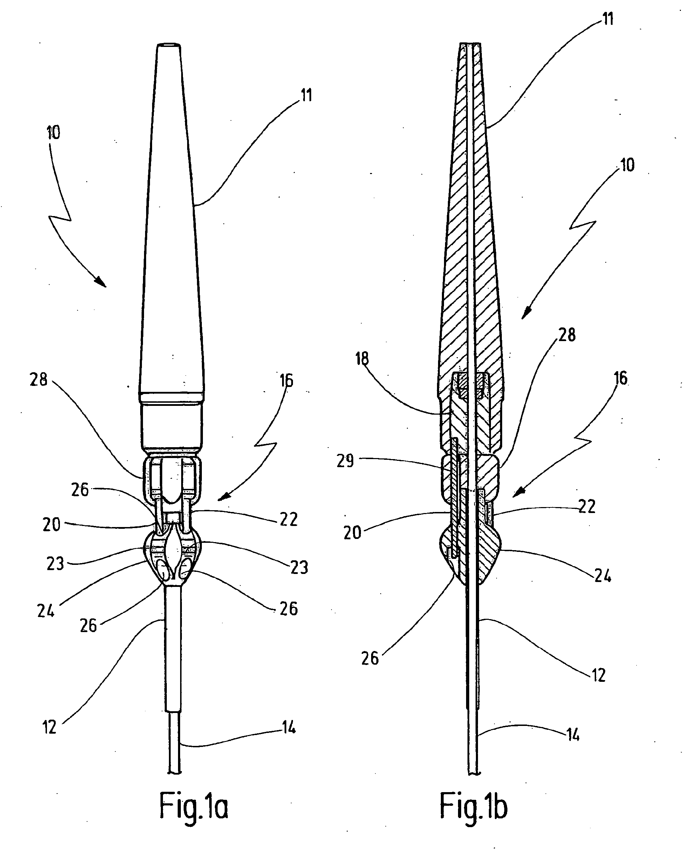

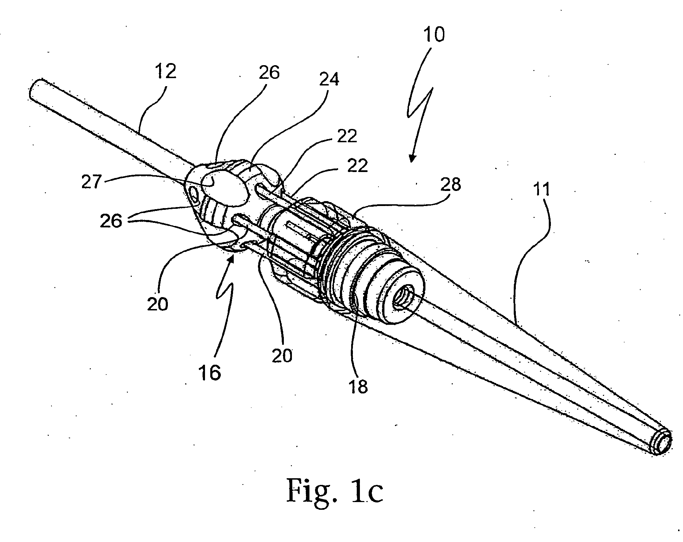

In den Fig. 1a, Fig. 1b und Fig. 1c ist mit 10 insgesamt ein erfindungsgemäßes Einführsystem bezeichnet, wobei in Fig. 1a der distale Abschnitt dieser Ausführungsform in seitlicher Draufsicht gezeigt ist, und in Fig. 1b der distale Abschnitt der Ausführungsform aus Fig. 1a in einem Längsschnitt. Fig. 1c zeigt eine seitliche Draufsicht des Ausführungsbeispiels aus den Fig. 1a und 1b. Dabei sind in den drei Figuren gleiche Elemente mit den gleichen Bezugszeichen versehen. Der Übersichtlichkeit halber ist der Stent sowie weitere übliche Merkmale eines Stenteinführsystems weggelassen worden.

Das Einführsystem, bei der eine Spitze 11 vorgesehen ist, weist ein äußeres Rohr 12 und ein inneres Rohr 14 auf. Ferner ist ein Fixiersystem vorgesehen, das in Fig. 1a, 1b und 1c insgesamt mit 16 gekennzeichnet ist. Das Fixiersystem 16 weist ein Deckelelement 18 auf, das wiederum Stiftelemente 20, 22 aufweist, die fest mit diesem verbunden sind. Ferner ist ein erstes Aufnahmeelement 24 vorgesehen, das Bohrungen 26 aufweist, in die die Stiftelemente 20, 22 in Fig. 1a, 1b und 1c eingeführt sind. Das erste Aufnahmeelement 24 weist auf seiner äußeren Oberfläche Vertiefungen 27 und dazwischen liegende Erhebungen 23 auf.

Das Deckelelement 18 ist dabei fest mit dem inneren Rohr 14 verbunden, das erste Aufnahmeelement 24 ist fest mit dem äußeren Rohr 12 verbunden. Wie Fig. 1b zu entnehmen ist, ist das Deckelsystem fest in der Spitze 11 des Einführsystems aufgenommen. Die Spitze 11 kann dabei aus einem weichen Material geformt sein.

Ferner ist ein zweites Aufnahmeelement 28 vorgesehen, das zwischen erstem Aufnahmeelement 24 und Deckelelement 18 angeordnet ist. Auch das zweite Aufnahmeelement 28 weit Bohrungen 29 auf, durch welche hindurch die im Deckelelement 18 befestigten Stiftelemente 20, 22 hindurchgeführt sind. Auch dieses zweite Aufnahmeelement 28 ist mit dem äußeren Rohr 12 fest verbunden.

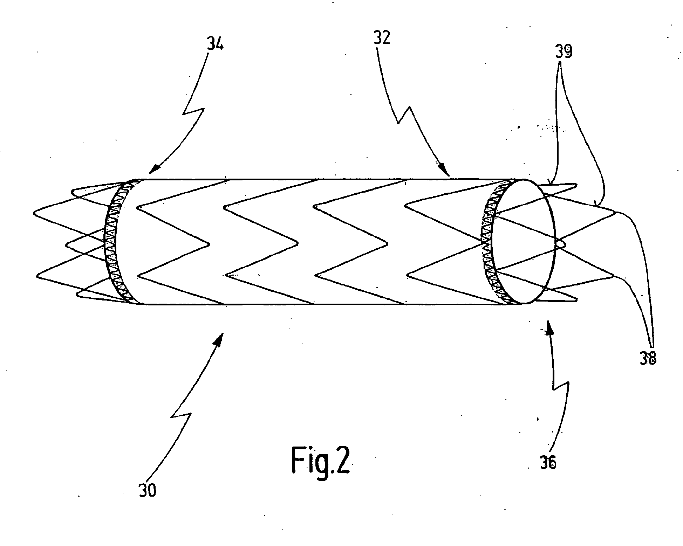

In Fig. 2 ist ein beispielhafter Stent 30 gezeigt, mit einem zylinderförmigen Hohlkörper, sowie einem proximalen 32 und einem distalen 34 Stentende. Das proximale Stentende 32 weist Spitzbögen auf, die in Fig. 2 insgesamt mit 36 gekennzeichnet sind. Diese Spitzbögen 36 weisen Scheitel 38 und gerade Abschnitte 39 auf.

Es versteht sich, dass der einzuführende Stent bzw. das proximale Stentende auch andere Formen als Spitzbögen aufweisen kann, wesentlich ist die maschenförmige Struktur des Stentendes, da mit dem erfindungsgemäßen Einführsystem bzw. Fixiersystem solche Maschen gegriffen und fixiert werden können.

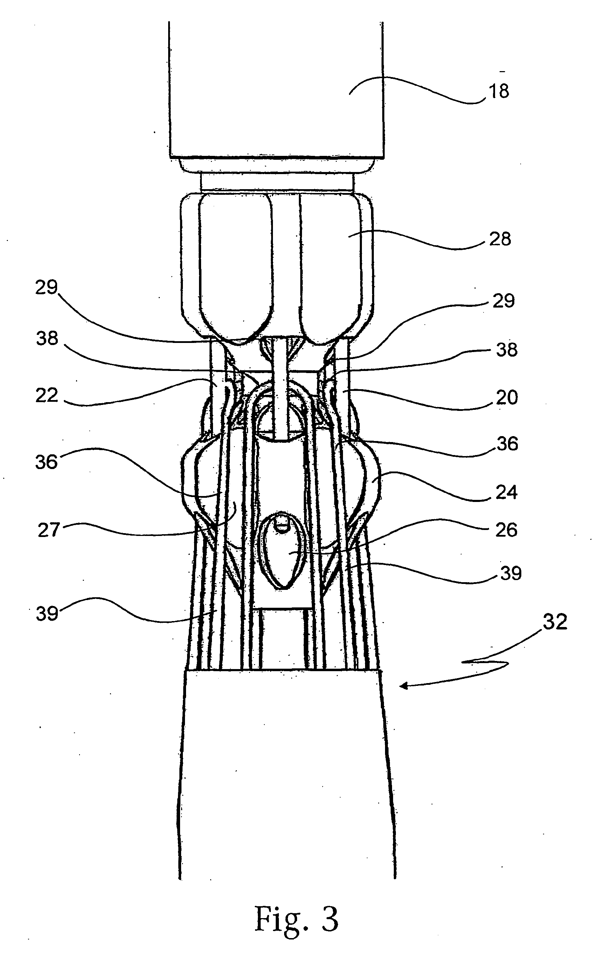

In Fig. 3 ist ein vergrößerter Ausschnitt einer Ausführungsform des erfindungsgemäßen Einführsystems dargestellt, wobei hier die gleichen Elemente wie in den Fig. 1a, b, c und 2 mit den gleichen Bezugszeichen versehen sind.

In Fig. 3 ist der distale Abschnitt eines Einführsystems 10 gezeigt. Dieser weist ein Fixiersystem 16 auf, welches ein Deckelelement 18 und Stiftelemente 20, 22, aufweist, die fest mit dem Deckelelement verbunden sind. In Fig. 3 ist ferner gezeigt, dass das Fixiersystem ein erstes Aufnahmeelement 24 umfasst, das Bohrungen 26 aufweist, in das die Stiftelemente 20, 22 eingeführt sind. Das erste Aufnahmeelement 24 weist ferner wie in Fig. 1 auf seiner äußeren Oberfläche Vertiefungen 27 auf. Mit 28 ist ein zweites Aufnahmeelement bezeichnet, das zwischen erstem Aufnahmeelement 24 und Deckelelement 18 angeordnet ist. Wie Fig. 3 zu entnehmen ist, weist auch das zweite Aufnahmeelement 28 Bohrungen 29 auf, durch welche die Stiftelemente 20, 22 hindurchgeführt sind.

Das zweite Aufnahmeelement 28 weist auf seiner Oberfläche aufgrund der Bohrungen 29 sich abwechselnde Erhebungen, in denen die Bohrungen 29 geführt sind, und Vertiefungen auf.

Ferner ist in Fig. 3 ein proximales Stentende 32 gezeigt, das Spitzbögen 36 mit Scheitel 38 und geraden Abschnitten 39 aufweist. Die geraden Abschnitte 39 der Spitzbögen 36 liegen dabei - also im geladenen Zustand des Einführsystems - in den Vertiefungen 27 des ersten Aufnahmeelements 24.

Wie in Fig. 3 gezeigt, greifen die Stiftelemente 20, 22, die im Deckelelement 18 befestigt sind, über die Bohrungen 29 durch das zweite Aufnahmeelement 28 hindurch. Ferner werden sie durch die Spitzbögen 36 hindurchgeführt, da deren Scheitel 38 aufgrund der Form des ersten Aufnahmeelements 24 von diesem abstehen und dadurch das Eingreifen der Stiftelemente 20, 22 in die Spitzbögen einfach ermöglicht wird. Die Stiftelemente 20, 22 sind ferner dann durch die Bohrungen 26 des ersten Aufnahmeelements 24 geführt, so dass die Spitzbögen 36 des Stents 30 fixiert sind.

Es versteht sich, dass die Form des ersten Aufnahmeelements 24 auch anders ausgebildet als in den Fig. abgebildet sein kann, wesentlich ist jedoch, dass die Form des ersten Aufnahmeelements der Form des proximalen Endes des einzusetzenden Stents bzw. den Maschen dieses Stents angepasst ist. Es versteht sich ferner, dass die Anzahl der Stiftelemente 20, 22 der Anzahl der Maschen oder Spitzbögen eines Stents entspricht, die mit dem Fixiersystem zur Einführung des Stents in ein Gefäß fixiert werden sollen.

Im Folgenden wird beschrieben, wie ein Stent auf das Einführsystem geladen wird: Um einen Stent 30 auf dem Einführsystem zur Einführung und Freisetzung des Stents in einem Körpergefäß anzubringen, wird dieser über das äußerer Rohr 12 geführt und mit seinem proximalen Stentende 32 über das erste Aufnahmeelement 24 hinweg. Dabei legen sich die geraden Abschnitte 39 der Spitzbögen 36 in die Vertiefung 27, die auf der Oberfläche des ersten Aufnahmeelements 24 vorgesehen sind. Das erste Aufnahmeelement 24 weist in seinem mittleren Abschnitt einen größeren Durchmesser auf als in seinen beiden Endabschnitten, und weist dadurch eine kugelige oder bauchige Form auf. Die Stiftelemente 20, 22 befinden sich in diesem Zustand nicht in den Bohrungen 26 des ersten Aufnahmeelements 24. Zur Fixierung des proximalen Stentendes 32 werden nun die Stiftelemente 20,22 durch ein Verschieben des inneren Rohres 14 und des damit fest verbundenen Deckelelements 18 durch das zweite Aufnahmeelement 28 hindurch geführt. Anschließend greifen die Stiftelemente 20, 22 mit ihren Enden in die Spitzbögen 36 und fädeln diese sozusagen auf. Mit den aufgefädelten Spitzbögen werden die Stiftelemente 20, 22 weiter in die Bohrungen 26 des ersten Aufnahmeelements 26 geführt und fixieren damit das proximale Stentende 32.

Die geraden Abschnitte 39 der Spitzbögen 36 legen sich dabei in Vertiefungen 27, die dazu vorgesehen sind, um das Einfügen der geraden Abschnitte 39 auf der Oberfläche des ersten Aufnahmeelements 24 zu ermöglichen. Dadurch, dass sich die geraden Abschnitte 39 derart "glatt" in die Vertiefungen 27 einfügen, wird vermieden, dass diese Abschnitte als Erhebungen auf der Oberfläche des ersten Aufnahmesystems 24 auftreten und eine unnötige Reibung verursachen.

Die übrigen Abschnitte des Stents sind von einer Rückzugshülle im komprimierten Zustand gehalten (nicht gezeigt). In diesem komprimierten und fixierten Zustand wird der Stent in ein Körpergefäß eingeführt. Hierzu ist üblicherweise auch eine Drahtführung vorgesehen, die durch das innere Rohr 14 und durch die Spitze 11 hindurch geführt ist. Über entsprechende Marker, bspw. Röntgenmarker, kann die genaue Platzierung des Stents verfolgt werden.

Wurde mit dem Einführsystem, bzw. mit dem darin gelagerten, komprimierten Stent, die Stelle erreicht, an welcher der Stent platziert werden soll, so wird das zunächst das innere Rohr 14 betätigt und nach vorne, in distale Richtung (vom Anwender weg), geschoben. Mit der Verschiebung des inneren Rohrs 14 in distale Richtung wird auch das mit diesem fest verbundene Deckelelement 18 in distale Richtung verschoben. Da die Stiftelemente 20, 22 fest mit dem Deckelelement 18 verbunden sind, werden auch sie durch die Verschiebung es inneren Rohrs 14 indirekt in distale Richtung verschoben. Dadurch werden sie aus den Bohrungen 26 des ersten Aufnahmeelements 24 herausgeführt, und geben die aufgefädelten Spitzbögen 36 des proximalen Stentendes 32 frei. Diese können dann expandieren und lagern sich an die Gefäßwand an. Die restlichen Abschnitte des Stents können bspw. durch Entfernen einer diesen komprimierenden Rückzugshülle vom Einführsystem 10 freigesetzt werden. Beim passiven Verschieben der Stiftelemente 20, 22 werden diese durch die Bohrungen 29 des zweiten Aufnahmeelement 28 geführt und bleiben mit ihren Enden in diesen enthalten. Dadurch wird ein Freiliegen von spitzen Enden vermieden, was insbesondere beim Rückziehen des inneren Rohres 14 durch den Stent 30 und durch das Einführungssystem 10 in Richtung Anwender hinaus von Vorteil ist, da damit nicht die Gefahr des Verhakens von freiliegenden spitzen Enden mit umgebenden Gewebe besteht.

Der auf dem äußeren Rohr 12 normalerweise gelagerte Stent 30 ist mit dem erfindungsgemäßen Einführsystem gegen Relativbewegungen bei der Freisetzung des proximalen Endes 32 geschützt, da lediglich das innere Rohr 14 bewegt wird, und das äußere Rohr 12 feststehend bleibt. Darüber hinaus sind durch das Fixiersystem 16 alle Teile gegen eine Rotation gesperrt, wodurch Drehmomente auf das System übertragen werden können.

## Patentansprüche

1. Einführsystem (10) zum Einführen eines selbstexpandierenden Stents in ein Körpergefäß, wobei das Einführsystem (10) folgendes aufweist:
einen selbstexpandierenden Stent (30) mit einem hohlzylindrischen Körper mit einem proximalen (32) und einem distalen (34) Ende, wobei zumindest das proximale (32) Ende abwechselnd in proximale und distale Richtung weisende Spitzbögen (36) aufweist, die einen Scheitel (38) und gerade Abschnitte (39) aufweisen,
ein äußeres Rohr (12), das innerhalb des Stents geführt ist, und ein inneres Rohr (14), das innerhalb des äußeren Rohrs (12) geführt ist, wobei das innere (14) und das äußere Rohr (12) gegeneinander axial verschiebbar angebracht sind, und
ein Fixiersystem (16) für die Spitzbögen (36) des proximalen Stentendes (32) zur Einführung des Stents (30) in das Körpergefäß, wobei das Fixiersystem (16) ein Deckelelement (18) mit daran befestigten Stiftelementen (20, 22) und ein erstes Aufnahmeelement (24) für die Stiftelemente (20, 22) aufweist, **dadurch gekennzeichnet, dass** sich die Stiftelemente (20, 22) axial und in proximaler Richtung des Einführsystems (10) erstrecken, dass das Aufnahmeelement (24) proximal zu dem Deckelelement (18) im Einführsystem (10) angeordnet ist, und dass das Deckelelement (18) fest mit dem inneren Rohr (14) verbunden ist, derart, dass durch eine Verschiebung des inneren Rohres (14) in distale Richtung die Spitzbögen (36) des proximalen Stentendes (32) aus dem Fixiersystem (16) freisetzbar und in einen expandierten Zustand überführbar sind.

2. Einführsystem (10) nach Anspruch 1, **dadurch gekennzeichnet, dass** das Fixiersystem (16) das erste Aufnahmeelement (24) für die in die proximale Richtung des Einführsystems (10) weisenden Stiftelemente (20, 22) fest mit dem äußeren Rohr (12) verbunden ist, derart, dass das erste Aufnahmeelement (24) und das Deckelelement (18) axial verschiebbar gegeneinander angeordnet sind.

3. Einführsystem (10) nach Anspruch 2, **dadurch gekennzeichnet, dass** das erste Aufnahmeelement (24) zumindest eine Bohrung (26) zur lösbaren Aufnahme der Stiftelemente (20, 22) aufweist.

4. Einführsystem (10) nach einem der Ansprüche 2 oder 3, **dadurch gekennzeichnet, dass** das erste Aufnahmeelement (24) längliche, in axiale Richtung zum Einführsystem (10) verlaufende Vertiefungen (27) auf dessen Oberfläche zur Aufnahme der gerade Abschnitte (39) der Spitzbögen (36) aufweist.

5. Einführsystem (10) nach einem der Ansprüche 2 bis 4, **dadurch gekennzeichnet, dass** das erste Aufnahmeelement (24) eine dem hohlzylindrischen Körper des Stents (30) angepasste Form aufweist, mit einem ersten, dem Deckelelement (18) zugewandten Ende und einem zweiten dem Deckelelement (18) abgewandten Ende, wobei zumindest das erste Ende des ersten Aufnahmeelements (24) einen kleineren Durchmesser aufweist als ein zwischen dem ersten und dem zweiten Ende gelegenes Mittelteil.

6. Einführsystem (10) nach einem der Ansprüche 2 bis 5, **dadurch gekennzeichnet, dass** das Fixiersystem (16) ferner ein axial zwischen Deckelelement (18) und erstem Aufnahmeelement (24) gelegenes zweites Aufnahmeelement (28) mit durchgehenden Bohrungen (29) zur Führung der Stiftelemente (20, 22) vorgesehen ist, welches in axialer Distanz zum ersten Aufnahmeelement (24) mit dem äußeren Rohr (12) fest verbunden ist, derart, dass das mit dem äußeren Rohr (!2) fest verbundene erste (24) und zweite (28) Aufnahmeelement gegen das mit dem inneren Rohr (14) fest verbundene Deckelelement (18) axial verschiebbar ist.

7. Einführsystem (10) nach Anspruch 6, **dadurch gekennzeichnet, dass** das zweite Aufnahmeelement (28) auf seiner Oberfläche längliche, in axiale Richtung zum Einführsystem verlaufende Vertiefungen und dazwischen liegenden Erhebungen aufweist.

8. Einführsystem (10) nach einem der Ansprüche 1 bis 7, **dadurch gekennzeichnet, dass** die Anzahl der Stiftelemente (20, 22) der Anzahl der Spitzbögen (36) des proximalen Stentendes (32) entspricht.

9. Einführsystem (10) nach einem der Ansprüche 1 bis 8, **dadurch gekennzeichnet, dass** mindestens zwei Stiftelemente (20, 22) vorgesehen sind.

10. Einführsystem (10) nach einem der Ansprüche 1 bis 9, **dadurch gekennzeichnet, dass** es ferner eine konisch geformte Spitze (11) aufweist, in der das Deckelelement (18) des Fixiersystems (16) gelagert ist.

11. Einführsystem (10) nach einem der Ansprüche 1 bis 10, **dadurch gekennzeichnet, dass** ferner eine Rückzugshülle vorgesehen ist, die den Stent (30) zur Einführung in ein Körpergefäß in einem komprimierten Zustand hält.

12. Einführsystem (10) nach einem der Ansprüche 1 bis 11, **dadurch gekennzeichnet, dass** ferner ein Schiebeelement vorgesehen ist, durch welches das distale Ende und der zwischen distalem und proximalen Ende liegende Teil des Stents in Verbindung mit einem Rückziehen der Rückzugshülle freisetzbar ist.

13. Einführsystem (10) nach einem der Ansprüche 1 bis 12, **dadurch gekennzeichnet, dass** das Einführsystem (10) insgesamt oder Teile davon aus einem oder mehreren röntgendichten Materialien hergestellt sind.

## Claims

1. Delivery system (10) for the introduction of a self-expanding stent into a body vessel, the delivery system (10) comprising:
a self-expanding stent (30) with a hollow cylindrical body and a proximal (32) and a distal (34) end, in which at least the proximal (32) end has projecting loops (36) pointing alternately in the proximal and distal direction, the loops having a shoulder (38) and straight sections (39),
an outer tube (12) which passes through the stent, and an inner tube (14) which passes through the outer tube (12), the inner tube (14) and the outer tube (12) being designed to move axially with respect to one another, and
a fixing system (16) for the projecting loops (36) at the proximal end of the stent (32) for introduction of the stent (30) into the body vessel, the fixing system (16) comprising a cap element (18) with pin elements (20, 22) fixed to it and a first engagement unit (24) for the pin elements (20, 22), **characterized in that** the pin elements (20; 22) point axially and in the proximal direction of the delivery system (10), that further the engagement unit (24) is positioned proximal with respect to the cap element (18) in the delivery system (10), and that the cap element (18) is fixed to the inner tube (14) in such a way that movement of the inner tube (14) in the distal direction can release the projecting loops (36) at the proximal end of the stent (32) from the fixing system (16) and allow them to expand.

2. The Delivery system (10) according to claim 1, **characterized in that**, in the fixing system (16), the first engagement unit (24) for the pin elements (20, 22) pointing in the proximal direction of the delivery system (10) is fixed to the outer tube (12) in such a way that the first engagement unit (24) and the cap element (18) can move axially with respect to one another.

3. The Delivery system (10) according to claim 2, **characterized in that** the first engagement unit (24) has at least one hole (26) for the releasable engagement of the pin elements (20, 22).

4. The Delivery system (10) according to either claim 2 or 3, **characterized in that** the first engagement unit (24) has long grooves (27) on its surface axial to the delivery system (10) to engage with the straight sections (39) of the projecting loops (36).

5. The Delivery system (10) according to anyone of claims 2 to 4, **characterized in that** the first engagement unit (24) has a shape adapted to the hollow cylindrical body of the stent (30) with one first end pointing towards the cap element (18) and a second end pointing away from the cap element (18), at least the first end of the first engagement unit (24) having a smaller diameter than a middle section between the first and second ends.

6. The Delivery system (10) according to anyone of claims 2 to 5, **characterized in that** the fixing system (16) also has a second engagement unit (28) positioned axially between the cap element (18) and the first engagement unit (24) with holes (29) passing through it to guide the pin elements (20, 22); the second engagement unit of which is fixed to the outer tube (12) at an axial distance from the first engagement unit (24) in such a way that the first (24) and second (28) engagement units fixed to the outer tube (12) can move axially with respect to the cap element (18) fixed to the inner tube (14).

7. The Delivery system (10) according to claim 6, **characterized in that** the second engagement unit (28) has long depressions on its surface in the axial direction relative to the delivery system with bulges between.

8. The Delivery system (10) according to anyone of claims 1 to 7, **characterized in that** the number of pin elements (20, 22) corresponds with the number of projecting loops (36) at the proximal end of the stent (32).

9. The Delivery system (10) according to anyone of claims 1 to 8, **characterized in that** it is provided with at least two pin elements (20, 22).

10. The Delivery system (10) according to anyone of claims 1 to 9, **characterized in that** it has a conical tip (11) which comprises the cap element (18) of the fixing system (16).

11. The Delivery system (10) according to anyone of claims 1 to 10, **characterized in that** it also has a retractable sleeve which holds the stent (30) in a compressed state for introduction into a body vessel.

12. The Delivery system (10) according to anyone of claims 1 to 11, **characterized in that** it also has a pusher which, in combination with a removal of the retractable sleeve, can release the distal end of the stent and the part of the stent between the distal end and the proximal end.

13. The Delivery system (10) according to anyone of claims 1 to 12, **characterized in that** the delivery system (10) as a whole or parts thereof is/are constructed of one or more radiopaque materials.

## Revendications

1. Système d'introduction (10) pour introduire un stent auto-expansible dans un vaisseau corporel, le système d'introduction (10) présentant les éléments suivants :
un stent auto-expansible (30) comportant un corps cylindrique creux doté d'une extrémité proximale (32) et d'une extrémité distale (34), au moins l'extrémité proximale (32) présentant des ogives (36) orientées alternativement dans la direction proximale et distale, qui présentent un sommet (38) et des segments rectilignes (39),
un tube externe (12) qui est conduit à l'intérieur du stent et un tube interne (14) qui est conduit à l'intérieur du tube externe (12), les tubes interne (14) et externe (12) étant montés de façon à pouvoir être décalés axialement l'un contre l'autre, et
un système de fixation (16) pour les ogives (36) de l'extrémité proximale du stent (32) pour insérer le stent (30) dans le vaisseau corporel, le système de fixation (16) présentant un élément de couvercle (18) avec des éléments en pointe (20, 22) fixés sur celui-ci et un premier élément de réception (24) pour les éléments en pointe (20, 22), **caractérisé en ce que** les éléments en pointe (20, 22) s'étendent axialement et en direction proximale du système d'introduction (10) de telle sorte que l'élément de réception (24) soit disposé sur le plan proximal par rapport à l'élément de couvercle (18) dans le système d'introduction (10), et **en ce que** l'élément de couvercle (18) est lié fixement avec le tube interne (14) de telle sorte que par un décalage du tube interne (14) dans la direction distale, les ogives (36) de l'extrémité proximale du stent (32) puissent être libérées du système de fixation (16) et puissent passer à un état déployé.

2. Système d'introduction (10) selon la revendication 1, **caractérisé en ce que** le système de fixation (16), le premier élément de réception (24) pour les éléments en pointe orientés dans la direction proximale du système d'introduction, est lié fixement au tube externe (12) de telle sorte que le premier élément de réception (24) et l'élément de couvercle (18) soient disposés de façon à pouvoir être décalés axialement l'un contre l'autre.

3. Système d'introduction (10) selon la revendication 2, **caractérisé en ce que** le premier élément de réception (24) présente au moins un alésage (26) pour la réception détachable des éléments en pointes (20, 22).

4. Système d'introduction (10) selon l'une des revendications 2 ou 3, **caractérisé en ce que** le premier élément de réception (24) présente des cavités (27) longitudinales s'étendant dans la direction axiale par rapport au système d'introduction (10) sur sa surface pour la réception des segments rectilignes (39) des ogives (36).

5. Système d'introduction (10) selon l'une des revendications 2 à 4, **caractérisé en ce que** le premier élément de réception (24) présente une forme adaptée au corps cylindrique creux du stent (30) avec une première extrémité tournée vers l'élément de couvercle (18) et une deuxième extrémité détournée de l'élément de couvercle (18), au moins la première extrémité du premier élément de réception (24) présentant un diamètre inférieur à une partie médiane située entre les première et deuxième extrémités.

6. Système d'introduction (10) selon l'une des revendications 2 à 5, **caractérisé en ce que** le système de fixation (16) est doté en outre d'un deuxième élément de réception (28) situé axialement entre l'élément de couvercle (18) et le premier élément de réception (24) comportant des alésages débouchants (29) pour l'introduction des éléments en pointe (20, 22), qui est relié fixement dans la distance axiale par rapport au premier élément de réception (24), au tube externe (12) de telle sorte que le premier (24) et le deuxième (28) éléments de réception puissent être décalés axialement en étant reliés fixement au tube externe (12) contre l'élément de couvercle (28) lié fixement au tube interne (24)

7. Système d'introduction (10) selon la revendication 6, **caractérisé en ce que** le deuxième élément de réception (28) présente sur sa surface des cavités longitudinales évoluant dans la direction axiale par rapport au système d'introduction et des proéminences situées entre elles.

8. Système d'introduction (10) selon l'une des revendications 1 à 7, **caractérisé en ce que** le nombre d'éléments en pointe (20, 22) correspond au nombre d'ogives (36) de l'extrémité proximale du stent (32).

9. Système d'introduction (10) selon l'une des revendications 1 à 8, **caractérisé en ce qu'**au moins deux éléments en pointe (20, 22) sont prévus.

10. Système d'introduction (10) selon l'une des revendications 1 à 9, **caractérisé en ce qu'**il présente en outre une pointe de forme conique (11) dans laquelle l'élément de couvercle (18) du système de fixation (16) est placé.

11. Système d'introduction (10) selon l'une des revendications 1 à 10, **caractérisé en ce que** de plus, une gaine de retrait est prévue, qui maintient le stent (30) dans un état comprimé pour l'introduction dans un vaisseau corporel.

12. Système d'introduction (10) selon l'une des revendications 1 à 11, **caractérisé en ce que** de plus, un élément poussoir est prévu, par le biais duquel l'extrémité distale et la partie du stent située entre les extrémités distale et proximale, en relation avec un retrait de la gaine de retrait peuvent être libérés.

13. Système d'introduction (10) selon l'une des revendications 1 à 12, **caractérisé en ce que** le système d'introduction (10) complet ou des parties de celui-ci sont fabriqués en un ou plusieurs matériaux radio-opaques.
